# EUROPEAN PATENT SPECIFICATION

(11) **EP 2 954 909 B1**
(45) Date of publication and mention of the grant of the patent: **16.10.2019**
(21) Application number: 14712056.2
(22) Date of filing: 06.02.2014
(51) Int. Cl.: A61M 1/02

(54) **AUTOMATIC DEVICE INTENDED FOR HOMOGENIZATION OF THE BLOOD BOTH IN THE TUBING AND STORAGE BAG**
VORRICHTUNG ZUR HOMOGENISIERUNG VON BLUT SOWOHL IM SCHLAUCH ALS AUCH IM BLUTBEUTEL
DISPOSITIF AUTOMATIQUE POUR L'HOMOGÉNÉISATION DU SANG DANS LA TUBULURE ET DANS LA POCHE DE STOCKAGE

(30) Priority: 07.02.2013 PT 10677113
(43) Date of publication of application: 16.12.2015
(73) Proprietor: Erofio Atlântico, S.A., 2440-373 Batalha (PT)
(72) Inventor: FONTES DE MELO PEREIRA, André, P-2495-553 Fátima (PT); SILVERIO SILVA, Edgar, P-2425-619 Monte Redondo LRA (PT); RIBEIRO DE JESUS, Fernando Miguel, P-2410-230 Leiria (PT); GONÇALVES MARTINHO, Pedro Miguel, P-2410-450 Leiria (PT); CARDOSO AGUIAR RODRIGUES, Ricardo, P-2440-339 Batalha (PT); MONTEIRO RUIVO, Tiago João, P-2440-386 Batalha (PT); MENDES RIBEIRO DE SOUSA ALVES, Maria Leopoldina, P-2410-021 Leiria (PT)
(74) Representative: Pereira da Cruz, Joao
(86) International application number: PCT/PT2014/000009
(87) International publication number: WO 2014/123437

(56) References cited:
- EP-A1- 1 693 072
- EP-A2- 0 639 384
- WO-A1-03/026724
- GB-A- 2 298 933
- JP-A- H01 131 644
- US-A1- 2002 143 244

## Description

This invention relates to an automatic device aimed at homogenizing the blood on the tubing and on the blood's storage bag. It shall be understood that in the scope of a blood transfusion, the blood to be administered to the patient must be previously tested in order to ensure that it presents the ideal conditions and is compatible with the receiver. The fact that the blood to be tested, and then administered, is stored in the bag and in the tubing comprising it, *à priori* requires the blood to be homogenized in such a way to ensure uniformity of all of its constituents so that the above mentioned test analysis is as accurate as possible. This invention now addresses the problem associated to the homogenization of the blood deposited in the tubing of the blood's storage bags, since the said homogenization is currently manually performed, as described and explained hereinafter. With this invention it will be possible to automatically execute the above mentioned procedure by using the device herein disclosed.

### Technical field of the invention and closest state of the art

Currently, the procedure for homogenizing the blood inside the tubing and storage bag is manually executed, occurring whenever there is the need to perform a blood transfusion. In order to homogenize the blood, a specific plier is currently used for that purpose, commonly known as milking plier, which presses the end of the tubing, thus pushing the blood into the bag under the action of human arm, i.e. the tubing is "squeezed" by the operator with the aid of a plier. The plier is then opened and the already homogenized blood is returned to the tubing. Following the eventual repetition of this operation, a small segment of the tubing is sealed and cut off, the respective blood fraction comprised in the said segment of the tubing being then extracted and taken for analysis.

According to the experts, the successive repetition of this manual hand-held process for the homogenization of blood, will, after a long period of time, cause injuries to healthcare professionals. The technical problem which this invention intends to solve is to be able to automatically execute the blood homogenization, without requiring human effort, i.e., allowing the elimination of human effort, but also improving the process for blood homogenization in itself. This invention was initially conceived by examining the operation of the aforementioned plier used in the manual process for the homogenization of blood, from which resulted the main ideas to address the issue of eliminating the human effort and improving the blood homogenization.

The first idea that came out of this was to create a machine that is similar to a home inkjet printer, wherein the blood bag is introduced in the device with the tubing fully stretched and a roller continually slides along the tubing with a movement which is identical to the one used in the manual process. However, this idea was soon set aside due to the fact that the bags could have a tubing of about 1 meter length or more, and that would require the development of an equipment of low compactness, thus causing the device to not be easily workable.

Subsequently, the idea was to develop a method in this study which was similar to the lamination process. With this solution, based upon an initial sketch, one came to the conclusion that it would be possible to develop a more compact equipment. Lamination is a process of plastic deformation, wherein the material is forced to pass between two (cylindrical) rollers which rotate in opposite directions, with the same peripheral velocity, and being apart from each other by a distance lower than the original thickness of the material to be deformed. The propulsion of the material during lamination occurs through friction forces which are generated between the rollers and the material to be deformed, although external tractive or compressive forces can also be applied. When passing between the two rollers, the material endures plastic and elastic deformation, in order to both reduce the original thickness and increase length and width.

This invention differs from the above described lamination process in as much as there is not a plastic deformation but only an elastic deformation. The tubing is made of a polymeric material, usually vinyl polychloride (PVC), which will be subject to compressive forces between rollers elastically only, thus keeping its mechanical properties.

A research on the state of art of the invention was made in order to ascertain the novelty of the same. Although said research has not found inventions which were similar to the one disclosed in this patent application, the closest state of the art is represented by the following patent documents: EP 0639384, US 2005/0196872; US 5110743; US 5160333 and US 2004/0032793. This invention differs from the inventions disclosed in the aforementioned patents because the blood homogenization process is performed automatically, using several mechanisms, such as: a roll system comprising a lower roller and an upper roller which are coupled to the relevant shafts. The lower roller is supported by bearings at each end and is coupled to a motor by means of a notched belt drive. The upper roller is also supported by bearings at each end, but it works as a moved shaft. To this roller is also attached a lifting system provided with two spindles, which are supported by bearings and shoes, so that it is axially moved through a stepper motor and using a sprocket drive. The roll system and the lifting system are actuated by means of a control mechanism including two sets of two switches *ON*/*OFF,* which allow to respectively control and monitor each system.

### Detailed description of the invention

The automatic device intended for homogenization of blood in the tubing and storage bag is comprised of:
1. a roll system which allows the tubing to be compressed as well as the longitudinal movement of the same under the effect of friction force generated between the rollers and the tubing, without the need to apply an external additional pull force. The roll system comprises the following:
   a. one upper roller (2), non-solidary with the upper shaft (6), provided with free rotational movement. The free rotational movement means that it is performed only by action of friction, with no motorisation being required for that purpose. The said roller includes a protruding collar which rests on the guide bar of the roll system. It is possible to additionally adapt two brakes (3) on each end of the upper roller (2) with the aim of preventing its transversal movement and keeping it aligned with the lower roller (1); and
   b. one lower roller (1), having a recessed collar and a geometry which allows it to fit, with due tolerance, over the ridge of the upper roller (2). This geometry was conceived in order to ensure that the tubing is guided during the blood homogenization process, as well as to avoid its displacement out of the device during its operation. It is comprised of a steel stud which attaches the lower roller (1) to the lower shaft (5) supporting it, in order to ensure that both will roll solidarily;
2. a lower shaft (5), coupled to the lower roller (1), connected to a driving belt (14) with two bearings (7), one at each end of the shaft (5), in order to minimize the attrition between the shaft (5) and the two shoes (8) resting over a support, the shoes holder (10). The absence of bearings (7) results in an increased wear between the shaft (5) and the shoe (8), which would lead to more frequent maintenance services; the said shaft (5) is a drive shaft;
3. a moved upper shaft (6), i.e. without motorisation, which is coupled to the upper roller (2);
4. an engine (9) and the respective transmission parts, namely two pulleys (11) and a driving belt (14), which provide the motorisation of the components. It is sustained by a support (12);
5. a lifting system, which allows the upper shaft (6) to be vertically moved, being provided with two endless spindles (25) (26) to perform this movement. To one of the spindles (25) is coupled a stepper motor (20), with the aid of a bolt (19), and which is attached to the outer chafer by means of fixing screws (18). Each spindle also includes a toothed wheel (21), these being connected to each other by a driving belt (15) for the accurate transmission of movement between the spindles (25) (26). Each spindle (25) (26) is provided with a slide (24) connecting it to the upper shaft (6);
6. three bearings (22) (23) which are arranged at the lower end of each spindle (25) (26), as well as at the upper end of the spindle (26), wherein each bearing is supported by shoes (27) (28), thus offering stability to the system, ensuring less friction and avoiding wear;
7. control system, which allows the system's movements to be automatically started, being comprised of four buttons which, once actuated, will allow the device's control operations to be executed:
   a. Two buttons control the movement of the lifting system's motor, one button to move it down (17a) and one button to move it up (17b).
   b. The other two buttons control the motorisation of both the upper and lower rollers, one ON button (16a) to switch on and one OFF button (16b) to switch off.

On the outside of the device, there are the following components:
- Two tubing guides (30) (31), which are laterally arranged in the device (one in each side) in order to guide the tubing during the homogenization process. This guiding not only allows the tubing to be guided towards the exit area, but it also prevents the said tubing from displacing along the existing space between the roller and the outer chafer intended for covering the device (29). It also avoids accidental injury to the user during operation of the device; and
- the flat support for the blood bag (33), which is aimed at sustaining the blood bag so that it remains conveniently accommodated during the homogenization process. The bag wherein the blood is stored may be provided with tubings of approximately 1 meter length, this being the reason why it would not be comfortable for the user to hold the bag while waiting for the homogenization process to be concluded. In order to solve this problem, the blood bag support (33) was mounted laterally, next to the guides (30) (31) and aligned with the contacting area of the lower (1) and upper (2) rollers, so that the tubing is not displaced.
- The front lid (34) and rear lid (35), the roller's chafing piece (32) as well as the base (36), all of them intended for the protection of the device.
- The fixing screws (13) for fastening both the shoes (28) and shoes holder (10) to the base (36).

As regards the lower roller (1) the material considered was steel, or other metallic or polymeric material, while for the lower shaft (5) it was steel or other metallic material. As regards the upper shaft (6) the material considered was also steel or other metallic material, while for the upper roller (2) it was a polymer (for instance, polytetrafluorethylene (PTFE) usually referred to by its trade name, Teflon) because it has a low friction coefficient, ensuring good rotation in the relevant shaft and reduced wearing. The said upper roller (2) can be made of another metallic material. In order to start and stop the functioning of the lower shaft (5) and its respective lower roller (1), two buttons are actuated on the front panel of the equipment (16a) (16b). The lifting system is actuated by means of an upward movement button (17b) and a downward movement button (17a).

Operation of the lifting system:
- The lower (1) and upper (2) rollers shall be initially put at a minimal distance, i.e., having enough space to allow the tubing to be adjustly positioned in the device, which corresponds to the zero position of the system;
- The motorisation is actuated so that the lower (1) and upper (2) rollers are brought together into a position, referred to as work position, which provides for leak tightness of the blood in the contact area between the lower (1) and upper (2) rollers and the tubing;
- This position is maintained until the process is finished, after which the system will return to the zero position, by actuation of the motorisation which promotes the withdrawal of the lower (1) and upper (2) rollers, until a new work cycle is initiated.

Operation of the control system:
- Starting from the zero position, the button which actuates the downward movement (17a) must be pressed. When the downward movement button of the lifting system (17a) is pressed, the system moves into the work position and remains there until the upward movement button (17b) is pressed, with the system being then restored to the zero position;
- After the correct positioning of the lower (1) and upper (2) rollers, the rotation movement is started (by pressing the (ON) button (16a)) and the tubing starts its longitudinal movement);
- The system remains in motion until the stop button (OFF) is pressed (16b);
- By pressing the OFF button (16b), the system will be motionless until the upward movement button (17b) of the upper roller (2) is pressed. For that purpose, the button (16b) uses the inverse logic, i.e., when in stand-by it will emit the ON signal, turning off when actuated.

As an important note, it shall be mentioned that the lifting system must be locked as soon as the start button (16a) of the rollers is pressed. This system shall be unlocked when the system is shut down by pressing the OFF button (16b). This will prevent the process from being inadvertently interrupted. In an emergency situation, or if a malfunction of any type is detected, the OFF button (16b) must be pressed.

### Description of the drawings

### Indication of the reference numbers:

1 - lower roller;
2 - upper roller;
3 - brake;
4 - steel stud;
5 - steel lower shaft;
6 - steel upper shaft;
7 - bearing;
8 - bearing shoes of the lower shaft;
9 - engine;
10 - shoe holder (8);
11 - pulley;
12 - engine support (9);
13 - fixing screw for fastening the components to the base;
14, 15 - driving belts;
16a - ON button of the roll system;
16b - OFF button of the roll system;
17a - downward button of the lifting system;
17b - upward button of the lifting system;
18 - engine's (9) fixing screw;
19 - bolt connecting the engine (9) to the spindle (25);
20 - stepper motor;
21 - toothed wheel;
22 - spindle's upper bearing (26);
23 - spindles lower bearings (25)(26);
24 - slide connecting the upper shaft (6) to the spindles (25) (26);
25, 26 - endless spindles;
27 - upper bearing shoe (22) of the spindle (26)
28 - lower bearings shoe (23) of the spindles (25) (26);
29 - outer chafer for covering the device;
30 - lateral guide of the tubing (entrance);
31 - lateral guide of the tubing (exit);
32 - chafing piece of the rollers (1) and (2);
33 - blood bag support;
34 - front lid;
35 - rear lid;
36 - base.

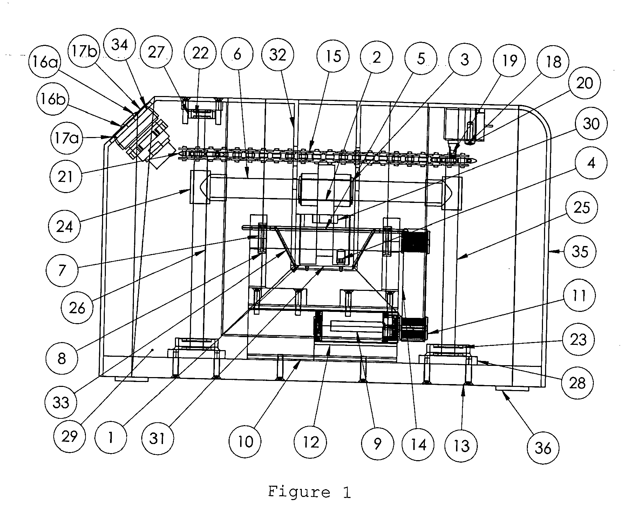

Figure 1 - Automatic device intended for homogenization of the blood on both the tubing and the bag, wherein all its components are depicted and which includes the following: the roll system (comprising the lower (1) and upper (2) rollers), the lower (5) and upper (6) shafts, the shoes (8) and the shoes holder (10), the bearings (7), the engine (9) and its support (12), the pulleys (11) and the driving belt (14), the steel stud (4) and the brakes (3). The lifting system is comprised of the spindles (25) and (26), the shoes (27) (28), the bearings (22) and (23), the motorisation and driving system such as the stepper motor (20), fixing screws (18) and bolt (19), gearings (21) and belt (15), and the slides (24). The control system is comprised of the two sets of buttons, the ON (16a) and OFF (16b) buttons of the roll system and the downward (17a) and upward (17b) buttons of the lifting system. These mechanisms are protected by the outer chafer (29) and the chafing piece of the rollers (32) as well as by the front lid (34) and the rear lid (35). It further comprises the base supporting the equipment (36), the blood bag support (33), the guides (30) and (31), and the fixing screws (13).

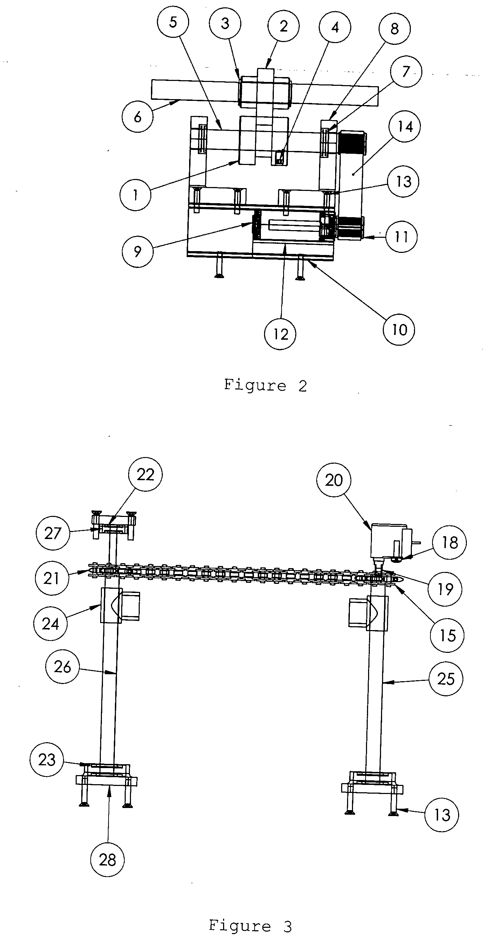

Figure 2 - Roll system comprised of: the lower (1) and upper (2) rollers, the lower (5) and upper (6) shafts, the supporting means such as the shoes (8) and the shoes holders (10), the bearings (7), the engine (9) and its support (12), the pulleys (11) and the driving belt (14), and also the steel stud (4), the brakes (3) and the fixing screws (13).
Figure 3 - Lifting system, wherein all its components are depicted, which includes the following: the spindles (25) (26), the shoes (27) (28) as supporting means, the bearings (22) (23), the stepper motor (20), the fixing screws (18) and bolts (19), the gearings (21) and the belt (15) and the slides (24), as well as the fixing screws (13) for fastening the components to the base (36)

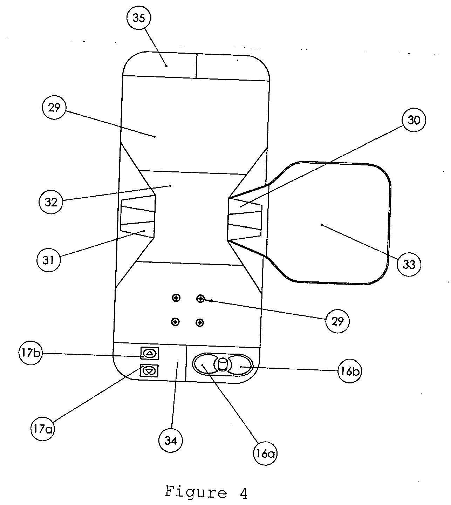

Figure 4 - This figure shows in detail the outer components of the device, namely: the guides (30) (31), the support (33) for the tubing, the control system comprised of the two sets of buttons, the ON (16a) and OFF (16b) buttons of the roll system and the downward (17a) and upward (17b) buttons of the lifting system, the front lid (34) and rear lid (35) and the outer chafer (29) and chafing piece of the rollers (32).

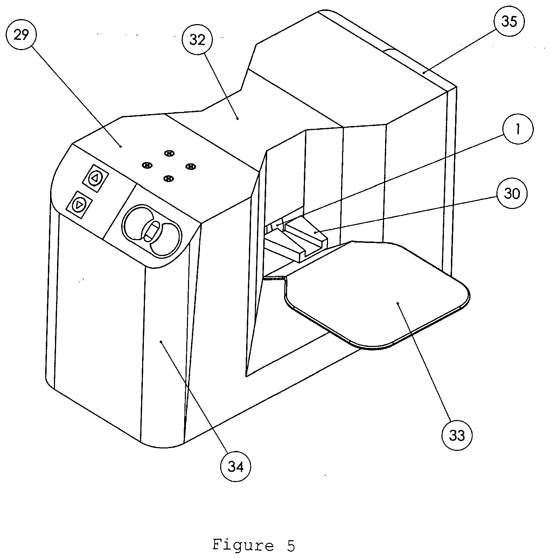

Figure 5 - This figure is an isometric view of the invention. It shows the outer components of the device, namely the front lid (34) and rear lid (35), the outer chafer (29) and the chafing piece of the rollers (32), the guides (30), the support (33) for the tubing and the lower roller (1).

## Claims

1. An automatic device intended for homogenization of blood both in a tubing and a storage bag, with an upper roller (2), non-solidary with the upper shaft (6), which includes a protruding collar, a lower roller (1) which is provided with a recessed collar and fits into the protruding collar of the upper roller (2), an upper shaft (6), coupled to the upper roller (2), an engine (9) and respective transmission parts, a supporting element (12), a control system comprised of four buttons, for the downward movement of the lifting system (17a), upward movement of the lifting system (17b) and controlling the lower and upper rollers, respectively, one for switch on (16a) and another for switch off (16b), **characterized in that** it comprises:
a) a steel stud attaching the lower roller (1) to the lower shaft (5), which is coupled to the lower roller (1) and connected to a driving belt (14) which is provided with two bearings (7), one at each end;
b) Two shoes (8) resting over a support, the shoes holder (10) ;
c) two endless spindles (25) (26), each one including a toothed wheel (21), these being connected to each other by a driving belt (15), and a slide (24) connecting it to the upper shaft (6);
d) three bearings (22) (23) which are arranged at the lower end of each spindle (25) (26), as well as at the upper end of the spindle (26), wherein each bearing is supported by shoes (27) (28).

2. A device according to claim 1 **characterized in that** the transmission parts of the engine (9) are two pulleys (11) and a driving belt (14).

3. A device according to claim 1 **characterized in that** the spindle (25) is coupled to a stepper motor (20) with the aid of a bolt (19), and attached to the outer chafer by means of fixing screws (18).

4. A device according to claim 1 **characterized in that** it comprises:
a) two guides (30) (31), which are laterally arranged on the outside of the device;
b) a flat support for the blood bag (33), which is mounted laterally on the outside, next to the guides (30) (31), and aligned with the contacting area between the lower (1) and the upper (2) rollers;
c) front lid (34) and rear lid (35), chafing piece of the rollers (32) and base (36);
d) fixing screws (13) for attaching the shoes (28) and the shoes holder (10) to the base (36).

5. A device according to claim 1 **characterized in that** the material of the lower roller (1) is steel, or other metallic or polymeric material.

6. A device according to claim 1 **characterized in that** the material of the lower shaft (5) is steel or other metallic material.

7. A device according to claim 1 **characterized in that** the material of the upper shaft (6) is steel or other metallic material.

8. A device according to claim 1 **characterized in that** the material of the upper roller (2) is a polymer or other metallic material.

## Patentansprüche

1. Ein automatisches Gerät, bestimmt für die Homogenisierung von Blut, sowohl in einem Rohr, als auch in einem Aufbewahrungsbeutel, mit einer oberen Walze (2) nicht solidarisch mit dem oberen Schaft (6), welcher einen vorstehenden Kragen umfasst, eine untere Walze (1), welche mit einem vertieften Kragen vorgesehen ist und in den vorstehenden Kragen der oberen Walze (2) passt, ein oberer Schaft (6) an die obere Walze (2) gekoppelt, ein Motor (9) und entsprechende Getriebeteile, ein Stützelement (12), ein Steuerungssystem, das vier Knöpfe umfasst, für die Abwärtsbewegung des Hubsystems (17a), Aufwärtsbewegung des Hubsystems (17b) und Steuerung der oberen und unteren Walzen, jeweils, eine für das Einschalten (16a) und eine andere für das Ausschalten (16b), **dadurch gekennzeichnet, dass** es folgendes umfasst:
a) ein Stahlbolzen, welcher die untere Walze (1) an den unteren Schaft (5) befestigt, welcher an die untere Walze (1) gekoppelt ist und mit einem Treibriemen (14) verbunden ist, welcher mit zwei Lagern (7) vorgesehen ist, einer an jedem Ende;
b) zwei Schuhe (8), die über einer Stütze aufliegen, die Schuhhalterung (10);
c) zwei endlose Spindeln (25)(26) jede umfasst ein Zahnrad (21), diese sind aneinander durch ein Triebriemen (15) verbunden, und eine Gleitbahn (24), die diesen an den oberen Schaft (6) verbindet;
d) drei Lager (22)(23), welche an dem unteren Ende jeder Spindel (25) (26) angeordnet sind, sowohl auch am oberen Ende der Spindel (26), worin jedes Lager von Schuhen (27) (28) gestützt wird.

2. Ein Gerät nach Anspruch 1, **dadurch gekennzeichnet, dass** die Getriebeteile des Motors (9) zwei Riemenscheiben (11) und ein Triebriemen (14) sind.

3. Ein Gerät nach Anspruch 1, **dadurch gekennzeichnet, dass** die Spindel (25) an einen Schrittmotor (20) mittels eines Bolzen (19) gekoppelt ist, und an den äußeren Scheuerschutz mittels Befestigungsschrauben (18) befestigt ist.

4. Ein Gerät nach Anspruch 1, **dadurch gekennzeichnet, dass** es folgendes umfasst:
a) zwei Führungen (30) (31), die seitlich an der Außenseite des Geräts angeordnet sind;
b) eine ebene Auflage für den Blutbeutel (33), welche seitlich an der Außenseite montiert ist, neben den Führungen (30) (31), und an der Kontaktfläche zwischen der unteren (1) und der oberen (2) Walzen ausgerichtet ist;
c) vorderen Deckel (34) und hinteren Deckel (35), Scheuerschutzteil der Walzen (32) und Basis (36);
d) Befestigungsschrauben (13), um die Schuhe (28) und die Schuhhalterung (10) an die Basis (36) zu befestigen.

5. Ein Gerät nach Anspruch 1, **dadurch gekennzeichnet, dass** das Material der unteren Walze (1) Stahl ist, oder ein anderes metallisches oder Polymermaterial ist.

6. Ein Gerät nach Anspruch 1, **dadurch gekennzeichnet, dass** das Material des unteren Schafts (5) Stahl oder ein anderes metallisches Material ist.

7. Ein Gerät nach Anspruch 1, **dadurch gekennzeichnet, dass** das Material des oberen Schafts (6) Stahl oder ein anderes metallisches Material ist.

8. Ein Gerät nach Anspruch 1, **dadurch gekennzeichnet, dass** das Material der oberen Walze (2) ein Polymer oder anderes metallisches Material ist.

## Revendications

1. Un dispositif automatique destiné à l'homogénéisation de le sang à la fois dans un tube et dans une poche de rangement, avec un rouleau supérieur (2) non solidaire de l'arbre supérieur (6) et qui comprend un collier saillant, un rouleau inférieur (1) qui est fourni avec un collier encastré et s'insère dans le collier en saillie du rouleau supérieur (2), un arbre supérieure (6) couplé au rouleau supérieur (2), un moteur (9) et des pièces de transmission respectives, un élément de support (12), un système de contrôle composé de quatre boutons, pour le mouvement descendant du système de levage (17a), mouvement ascendant du système de levage (17b), et pour contrôler les rouleaux inférieur et supérieur, respectivement un pour allumer (16a) et un autre pour éteindre (16b), **caractérisé en ce qu'**il comprend:
a) un goujon en acier fixant le rouleau inférieur (1) au arbre inférieur (5), laquelle est couplée au rouleau inférieur (1) et reliée à une courroie de transmission (14) qui est munie de deux paliers (7), un à chaque extrémité;
b) deux chaussures (8) reposant sur un support, le porte-chaussures (10);
c) deux broches sans fin (25) (26), chacun comprenant un roue dentée (21), reliées entre elles par une courroie de transmission (15), et une glissière (24) les reliant au arbre supérieure (6);
d) trois paliers (22) (23) qui sont disposés à l'extrémité inférieure de chaque broche (25) (26), ainsi qu'à l'extrémité supérieure de la broche (26), dans laquelle chaque palier est soutenu par des chaussures (27) (28).

2. Un dispositif selon la revendication 1, **caractérisé en ce que** les pièces de transmission du moteur (9) sont deux poulies (11) et une courroie de transmission (14) .

3. Un dispositif selon la revendication 1, **caractérisé en ce que** la broche (25) est couplée à un moteur pas à pas (20) avec l'aide d'un goujon (19), et fixé au tablier extérieur par des moyens de fixation à vis (18).

4. Un dispositif selon la revendication 1, **caractérisé en ce qu'**il comprend:
a) deux guides (30) (31) disposés latéralement sur l'extérieur du dispositif;
b) un support plat pour la poche de sang (33), qui est monté latéralement à l'extérieur, à côté des guides (30) (31), et aligné avec la zone de contact entre les rouleaux inférieur (1) et supérieur (2);
c) couvercle avant (34) et couvercle arrière (35), pièce de tablier des rouleaux (32) et base (36);
d) des vis de fixation (13) pour fixer les chaussures (28) et le porte-chaussures (10) à la base (36).

5. Un dispositif selon la revendication 1, **caractérisé en ce que** le matériau du rouleau inférieur (1) est en acier ou autre matériau métallique, ou en polymère.

6. Un dispositif selon la revendication 1, **caractérisé en ce que** le matériau de l'arbre inférieur (5) est acier ou un autre matériau métallique.

7. Un dispositif selon la revendication 1, **caractérisé en ce que** le matériau de l'arbre supérieur (6) est acier ou un autre matériau métallique.

8. Un dispositif selon la revendication 1, **caractérisé en ce que** le matériau du rouleau supérieur (2) est un polymère, ou un autre matériau métallique.
